# EUROPEAN PATENT SPECIFICATION

(11) **EP 1 864 655 B1**
(45) Date of publication and mention of the grant of the patent: **30.11.2016**
(21) Application number: 06728832.4
(22) Date of filing: 09.03.2006
(51) Int. Cl.: A61K 31/192, A61K 9/70, A61K 47/32, A61K 47/44, A61L 15/58, C09J 153/02, C08K 5/01, C08L 23/22, C08K 5/103

(54) **ADHESIVE AND ADHESIVE PATCH**
KLEBSTOFF UND KLEBEPFLASTER
ADHESIF ET TAMPON ADHESIF

(30) Priority: 10.03.2005 US 660065 P; 10.03.2005 JP 2005067750
(43) Date of publication of application: 12.12.2007
(73) Proprietor: Hisamitsu Pharmaceutical Co., Inc., Tosu-shi, Saga 841-0017 (JP)
(72) Inventor: HIRANO, Munehiko, rodaikan-machi, Tosu-shi, Saga, 8410017 (JP); SHINMURA, Miyuki, rodaikan-machi, Tosu-shi, Saga, 8410017 (JP); TSURUDA, Kiyomi, rodaikan-machi, Tosu-shi, Saga, 8410017 (JP)
(74) Representative: Grünecker Patent- und Rechtsanwälte PartG mbB
(86) International application number: PCT/JP2006/304615
(87) International publication number: WO 2006/095820

(56) References cited:
- EP-A1- 1 366 762
- WO-A1-01/68061
- WO-A1-01/78690
- WO-A1-01/95889
- WO-A1-93/04677
- WO-A1-96/08245
- WO-A1-98/24423
- JP-A- 2004 043 512

## Description

### Technical Field

The present invention relates to an adhesive and plaster, and more specifically it relates to an adhesive and plaster comprising ketoprofen.

### Background Art

Anti-inflammatory analgesic plasters are known which are prepared by adding a non-steroidal anti-inflammatory analgesic, together with a dissolving agent (composed of a rosin ester derivative and L-menthol) to a styrene-isoprene-styrene block copolymer-based adhesive (Japanese Patent Publication No. 2816765).

In the plaster described in above mentioned Patent document, however, reaction often occurs between the L-menthol and the drug during production or during storage, while the characteristic odor of L-menthol is also unpleasant for many patients, and therefore research has been conducted for plasters that exhibit suitable drug release properties without addition of L-menthol. One such plaster that has been proposed in recent years is a styrene-isoprene-styrene block copolymer-based adhesive containing added prescribed amounts of high-molecular-weight polyisobutylene, low-molecular-weight polyisobutylene, and a dispersing agent and the like (International Publication WO01/078690 Pamphlet).

Other relevant prior art documents are EP1366762A1 and EP1293199A1.

### Disclosure of the Invention

### Problems to be Solved by the Invention

A demand has long existed for a plaster which has higher drug absorption efficiency and more excellent adhesion than the prior art and which is resistant to skin irritation, while employing a system that does not use L-menthol, and the demand has been particularly strong for a plaster using ketoprofen as the drug. It is therefore an object of the present invention to provide a ketoprofen-containing adhesive and plaster which contain no L-menthol, and which have high percutaneous absorption, excellent adhesion to skin and skin irritation reduced to an acceptable level.

As a result of much diligent research directed toward achieving the aforestated object, the present inventors have discovered that the object can be achieved if prescribed components, namely a base with tackiness and an oil that is compatible with the component, are added in a prescribed ratio when using ketoprofen as the drug in a system employing no L-menthol.

Specifically, the invention provides an adhesive comprising a base with tackiness, an oil and ketoprofen, wherein said adhesive contains no L-menthol, said base is composed at least of a tacky composition comprising an elastomer and a tackifier, said oil is an oil that is compatible with said tacky composition, and the content of said oil is 150-175 parts by weight with respect to 100 parts by weight as the total of said tacky composition, wherein said elastomer is a styrene-isoprene-styrene block copolymer with a styrene content of 15-25 wt% and a diblock content of no greater than 20 wt%, and wherein the mixing proportion of the tackifier is 50-200 parts by weight with respect to 100 parts by weight of the elastomer.

The adhesive of the invention is characterized by having a construction employing a prescribed amount of oil which is compatible with the tacky composition comprising an elastomer and a tackifier, and with the tacky polymer containing an unsaturated monomer with a total of 5 or more carbon atoms as the monomer unit, whereby it is possible to achieve high percutaneous absorption, excellent adhesion to skin and effective prevention of skin irritation. In addition, stratum corneum detachment is prevented and thus pain during peel-off is reduced.

While the cause of this effect is not completely understood, it is conjectured that the prescribed amount of oil used in excess of that which is commonly used results in moderate softening of the base with tackiness, thereby increasing adhesion with the skin and providing adequate percutaneous absorption properties. The moderate softening of the base with tackiness increases the shape-matching property on skin during use while facilitating deformation of the adhesive during peel-off, thereby allowing both improved adhesion and decreased pain during peel-off. Furthermore, the presence of the oil at the interface between the adhesive and the skin reduces the required peel-off force and protects the stratum corneum, so that detachment of the stratum corneum during peel-off is minimized and pain is reduced, thereby also preventing skin irritation.

Using the styrene-based block copolymer as the elastomer of the tacky composition can result in notable improvements in percutaneous absorption, adhesion to skin and prevention of skin irritation.

Suitable tacky polymers containing an unsaturated monomer with a total of 5 or more carbon atoms as the monomer unit are tacky polymers containing an alkyl (meth)acrylate with 4-22 carbon atoms in the alkyl group as the monomer unit. A "(meth)acrylate" is an acrylate or methacrylate.

The oil which is compatible with the tacky composition and tacky polymer is preferably at least one selected from the group consisting of almond oil, olive oil, camellia oil, persic oil, peanut oil and liquid paraffin. Using such an oil not only increases the percutaneous absorption but also results in excellent improvement in skin adhesion and prevention of skin irritation.

The ketoprofen content is preferably 0.5-3 wt% based on the total weight of the adhesive. This range for the content will permit percutaneous absorption of an adequate amount of drug.

The ketoprofen content is more preferably 2 wt% based on the total weight of the adhesive. Such content will result in a sufficient blood level of ketoprofen.

The adhesive may also comprise a support. In other words, the effects of the invention (high percutaneous absorption, excellent adhesion to skin, effective prevention of skin irritation , reduced pain during peel-off due to minimal stratum corneum detachment) are also exhibited by a plaster provided with the support bearing a drug layer comprising the aforementioned adhesive (adhesive with support).

In order to more reliably exhibit these effects, the drug layer is preferably formed on the support in an amount for coating of 80-210 g/m². The support may be made of a polyester, polyethylene or polypropylene, and is preferably a woven fabric or nonwoven fabric with a weight of 80-150 g/m². Here, "woven fabric" also includes knitted fabrics.

Since the adhesive of the invention has excellent percutaneous absorption, an area under the blood concentration-time curve at 0-72 hours (AUC₍₀₋₇₂ₕᵣ₎) of 1000-3000 ng·hr/mL can be achieved for ketoprofen upon contact with skin, and a maximum blood concentration (Cₘₐₓ) of 50-150 ng/mL can be achieved for ketoprofen upon contact with skin.

The plaster of the invention is a plaster provided with a support bearing a drug layer comprising an adhesive, wherein the adhesive comprises a base with tackiness, an oil and ketoprofen, the adhesive contains no L-menthol, the base is composed at least of a tacky composition comprising an elastomer and a tackifier and/or a tacky polymer containing an unsaturated monomer with a total of 5 or more carbon atoms as the monomer unit, the oil is an oil that is compatible with the tacky composition and the tacky polymer, the support consists of a woven fabric or nonwoven fabric comprising at least one type of resin fiber selected from the group consisting of polyester-based resins, polyethylene-based resins and polypropylene-based resins, the area of the drug layer to be contacted with the skin is 50-150 cm², and the area under the blood concentration-time curve at 0-72 hours (AUC₍₀₋₇₂ₕᵣ₎) for ketoprofen upon contact of the drug layer with skin is 1000-3000 ng·hr/mL.

The plaster of the invention preferably has a maximum blood concentration (Cₘₐₓ) of 50-150 ng/mL for ketoprofen upon contact of the drug layer with skin.

### Effect of the Invention

The invention provides a ketoprofen-containing plaster which contain no L-menthol, and which have high percutaneous absorption, excellent adhesion to skin and skin irritation reduced to an acceptable level.

### Best Modes for Carrying Out the Invention

Preferred embodiments of the adhesive and plaster of the invention will now be described in detail.

The adhesive of the invention is an adhesive comprising a base with tackiness, an oil and ketoprofen, and containing no L-menthol.

The lack of L-menthol in the adhesive prevents esterification of drugs that occurs when L-menthol coexists with carboxyl group-containing drugs such as ketoprofen. Thus, the quantity of the drug that penetrates the skin and actually functions is not reduced, thereby increasing the effective release dose of the drug. Furthermore, because esterification does not occur, there is no need to add esterification inhibitors such as fatty acid metal salts, thus permitting a more versatile formulation to be produced.

The "ketoprofen" mentioned above includes its pharmaceutically acceptable salts. As specific examples of pharmaceutically acceptable salts there may be mentioned sodium salts, potassium salts and diethylamine salts.

Because the adhesive is used by attachment to the skin, the base with tackiness (hereinafter also referred to simply as "base") is an essential component. Here, "tackiness" means that it exhibits tack at least at a moderate temperature (temperature for attachment to skin), and the elastic modulus E' at 1 Hz at that temperature may be 0.1 MPa or lower (Dahlquist's criterion). The base may be a single type or a combination of two or more types so long as it exhibits the aforementioned property. As typical bases there may be mentioned tacky compositions obtained by adding a tackifying resin to an elastomer, and tacky polymers containing an unsaturated monomer with 5 or more carbon atoms as the monomer unit. The term "elastomer" refers to any non-polyisobutylene elastomer, regardless of the molecular weight.

As tacky compositions there may be mentioned tacky compositions that exhibit tackiness by addition of tackifiers to natural rubber and tacky compositions that exhibit tackiness by addition of tackifiers to synthetic rubber, as well as blends thereof. Tacky compositions that exhibit tackiness by addition of tackifiers to synthetic rubber are preferred for ease of formulation. Preferred compositions of this type are those obtained by addition of tackifiers to thermoplastic elastomers, and particularly preferred are tacky compositions obtained by addition of tackifiers to styrene-based block copolymers.

As styrene-based block copolymers there may be mentioned styrene-butylene-styrene block copolymer (SBS), styrene-isoprene-styrene block copolymer (SIS), styrene-ethylene/butylene-styrene block copolymer (SEBS) and styrene-ethylene/propylene-styrene block copolymer (SEPS). These styrene-based block copolymers may have linear, diblock, radial or star backbones, but linear is preferred. A linear copolymer may partially include a diblock copolymer in the synthesis pathway, and therefore linear copolymers partially containing diblock copolymers may be used. The styrene-based block copolymer used may be of a single type or a combination of two or more types.

As specific examples of styrene-based block copolymers there may be mentioned linear triblock copolymers such as KRATON D-1112, D-1111 and D-1107 (trade names of Kraton Polymers), JSR5000 and JSR5002 (trade names of JSR Corp.), QUINTAC 3530, 3421 and 3570C (trade name of Zeon Corp.) and KRATON D-KX401CS and D-1107CU (trade name of Kraton Polymers), and branched block copolymers such as KRATON D-1124 (trade name of Kraton Polymers) and SOLPRENE 418 (trade name of Phillips Petroleum Co.)

The styrene-isoprene-styrene block copolymer has a styrene content of 15-25 wt% and a diblock content of no greater than 20%. By using such a styrene-based block copolymer it is possible to achieve more excellent percutaneous absorption and adhesion to skin, while reducing skin irritation.

The high styrene content of 20 wt% or greater and the low diblock content of no greater than 20 wt% improves the hardness of the styrene-based block copolymer, while also improving the hardness of the tacky composition obtained by admixing with the tackifier, but by using a hard styrene-based block copolymer and a higher content of oil than according to the prior art, more notable improvement can be achieved in the percutaneous absorption, adhesion to skin and prevention of skin irritation, compared to using a soft styrene-based block copolymer with addition of a small amount of oil.

The tackifier is not particularly restricted so long as it is a resin that can impart tackiness to the elastomer, but rosin-based resins and petroleum-based resins are preferred.

As rosin-based resins there may be mentioned natural resin rosins, modified rosins, rosin esters (rosin glycerin esters, rosin pentaerythritol esters, etc.) and hydrogenated rosin esters (hydrogenated rosin glycerin esters, hydrogenated rosin pentaerythritol esters, etc.), among which hydrogenated rosin esters are preferred and hydrogenated rosin glycerin esters are particularly preferred, from the standpoint of low stimulation of the skin and aging resistance.

As petroleum-based resins there may be mentioned C5-based synthetic petroleum resins (copolymers of at least two from among isoprene, cyclopentadiene, 1,3-pentadiene and 1-pentene; copolymers of at least two from among 2-pentene and dicyclopentadiene; resins composed mainly of 1,3-pentadiene, etc.), C9-based synthetic petroleum resins (copolymers of at least two from among indene, styrene, methylindene and α-methylstyrene) and dicyclopentadiene-based synthetic petroleum resins (copolymers of isoprene and/or 1,3-pentadiene composed mainly of dicyclopentadiene, etc.), but C9-based synthetic petroleum resins are preferred from the viewpoint of weather resistance and compatibility with the base with tackiness.

From a different viewpoint of classification of petroleum-based resins there may be mentioned alicyclic petroleum resins (alicyclic hydrocarbon resins), alicyclic hydrogenated petroleum resins, aliphatic petroleum resins (aliphatic hydrocarbon resins), aliphatic hydrogenated petroleum resins, aromatic petroleum resins and the like, among which alicyclic petroleum resins and alicyclic hydrogenated petroleum resins are preferred and alicyclic hydrogenated petroleum resins are particularly preferred, from the standpoint of strength of tackiness, compatibility with the base with tackiness and aging resistance. As specific petroleum resins there may be mentioned ARKON-P70, ARKON-P90, ARKON-P100, ARKON-P115 and ARKON-P125 (trade names of Arakawa Chemical Co., Ltd.) and ESCOREZ 8000 (trade name of Esso Petroleum Co.), which may be used alone or in combinations of two or more.

The mixing ratio of the elastomer and the tackifier may be determined so as to exhibit tack at least at moderate temperature (temperature for attachment to skin). A typical mixing proportion of the tackifier is 10-1000 parts by weight (preferably 50-200 parts by weight, more preferably 50-150 parts by weight and even more preferably 80-120 parts by weight) with respect to 100 parts by weight of the elastomer.

A tacky polymer containing an unsaturated monomer with a total of 5 or more carbon atoms as the monomer unit may also be used as the base in addition to the tacky composition comprising the elastomer and tackifier. As tacky polymers containing an unsaturated monomer with a total of 5 or more carbon atoms as the monomer unit there may be mentioned tacky polymers containing an alkyl (meth)acrylate with 4-22 carbon atoms in the alkyl group as the monomer unit, and such polymers have tackiness even without addition of a tackifier. As alkyl (meth)acrylates with 4-22 carbon atoms in the alkyl group there may be mentioned butyl (meth)acrylate, hexyl (meth)acrylate, octyl (meth)acrylate, 2-ethylhexyl (meth)acrylate, decyl (meth)acrylate, dodecyl (meth)acrylate, tetradecyl (meth)acrylate, hexadecyl (meth)acrylate and octadecyl (meth)acrylate. A tacky polymer containing an unsaturated monomer with a total of 5 or more carbon atoms as the monomer unit may be used alone, or two or more different types may be used in combination.

The tacky polymer is preferably a copolymer containing not only an alkyl (meth)acrylate with 4-22 carbon atoms in the alkyl group, but also other monomers as a monomer unit. As other such monomers there are preferred monomers which have a glass transition temperature of 50°C or higher (measured by DSC with 5°C temperature increase) when homo-polymerized. As such monomers there may be mentioned (meth)acrylic acid, methyl (meth)acrylate, isobornyl (meth)acrylate, N-vinylpyrrolidone and styrene.

In the case of a copolymer, the weight ratio of the alkyl (meth)acrylate with 4-22 carbon atoms in the alkyl group and the other monomer may be set so that either the glass transition temperature (Tg) as calculated by the Fox's equation is below the temperature at which the adhesive is used (typically room temperature of about 25°C), or so that measurement of the dynamic viscoelasticity of the polymer (at a frequency of about 1 Hz) gives a storage elastic modulus on the order of 10⁶ dyne/cm² at the temperature at which the adhesive is used (typically room temperature, about 25°C); however, generally the weight ratio of the former:latter weight ratio will be 90-98:2-10.

The base may comprise more than one component. For example, it may be a blend comprising two or more different types of tacky compositions each composed of an elastomer and a tackifier, or a blend comprising two or more different tacky polymers each containing an unsaturated monomer with a total of 5 or more carbon atoms as the monomer unit. It may also be a blend of a tacky composition comprising an elastomer and tackifier, and a tacky polymer containing an unsaturated monomer with a total of 5 or more carbon atoms as the monomer unit. In such cases, the blend ratio (weight ratio) of former:latter is preferably 10-90:90-10 and more preferably 20-80:80-20. The base may also contain tacky components other than the aforementioned tacky composition and tacky polymer.

The adhesive contains an oil that is compatible with the tacky composition and the tacky polymer, the oil content being 150-175 parts by weight with respect to 100 parts by weight as the total of the tacky composition and/or tacky polymer.

As such oils there may be used one or more selected from the group consisting of almond oil, olive oil, camellia oil, persic oil, peanut oil and liquid paraffin, and liquid paraffin is especially preferred for use.

The oil content is determined solely with respect to the "tacky composition composed of an elastomer and tackifier" and/or the "tacky polymer containing an unsaturated monomer with a total of 5 or more carbon atoms as the monomer unit" which are present in the base. For example, when the base contains other compounds (such as polyisobutylene containing an unsaturated monomer with a total of 4 carbon atoms as the monomer unit), they are not used in calculation of the oil content even if they have tackiness.

The present invention has been accomplished on the basis of the new knowledge that, for a adhesive containing ketoprofen but no L-menthol, the percutaneous absorption, adhesion to skin and skin irritation are all improved when an oil is used at 150-175 parts by weight with respect to 100 parts by weight as the total of the "tacky composition comprising an elastomer and a tackifier" and "tacky polymer containing an unsaturated monomer with a total of 5 or more carbon atoms as the monomer unit". Specifically, an oil content of less than 150 parts by weight results in reduced drug release properties and percutaneous absorption, while a content of greater than 175 parts by weight tends to result in stickiness, reduces adhesion to the skin and leads to cohesion failure of adhesive during peel-off. The oil content is more preferably 150-170 parts by weight and most preferably 155-165 parts by weight for optimization of the aforementioned properties.

The following is a preferred total composition for the adhesive. First, ketoprofen is preferably included at 0.5-3 wt%, more preferably 1-2.5 wt% and even more preferably 2 wt% based on the total weight of the adhesive. While addition of drugs other than ketoprofen is not prohibited, the drug effect of ketoprofen must be maintained. The total weight of the base with tackiness and the oil is preferably 80-100 wt% (more preferably 90-100 wt% and even more preferably 95-99 wt%) of the remainder after subtracting the ketoprofen content from the adhesive content. If the value is 100 wt%, the adhesive will be composed only of the base with tackiness, the oil and ketoprofen, and when it is less than 100 wt%, it will contain other added components described below. The base with tackiness preferably is composed only of the "tacky composition comprising an elastomer and a tackifier" and/or the "tacky polymer containing an unsaturated monomer with a total of 5 or more carbon atoms as the monomer unit", and more preferably it consists only of the "tacky composition comprising an elastomer and a tackifier" since this will facilitate mixing and allow mixing without a solvent.

According to the invention, the adhesive contains a base with tackiness, an oil and ketoprofen, while containing no L-menthol, and the base is composed of a tacky composition comprising an elastomer and a tackifier, and/or a tacky polymer containing an unsaturated monomer with a total of 5 or more carbon atoms as the monomer unit, while the oil is compatible with the tacky composition and the tacky polymer, so that the ketoprofen can exist in a dissolved state in the adhesive.

Generally speaking, a dissolving agent is necessary for a drug to exist in a dissolved state in an adhesive. In the past, L-menthol or the like has functioned as a dissolving agent for ketoprofen-containing plasters. According to the invention, however, it was found surprisingly that ketoprofen can exist in a dissolved state in an adhesive through the effect of the aforementioned elastomer and oil, without adding a dissolving agent.

Furthermore, regardless of the dissolved state, the conditions may be set for an area under the blood concentration-time curve at 0-72 hours (AUC₍₀₋₇₂ₕᵣ₎) of 1000-3000 ng·hr/mL for ketoprofen upon contact of the adhesive with skin.

As non-essential components that may be added to the adhesive there may be mentioned antioxidants, fillers, crosslinking agents, antiseptics, ultraviolet absorbers, absorption accelerators and various polymers (tacky and non-tacky).

As antioxidants there are preferred tocopherol and its ester derivatives, ascorbic acid, ascorbyl stearic acid esters, nordihydroguaiaretic acid, dibutylhydroxytoluene and butylhydroxyanisole.

As fillers there are preferred calcium carbonate, magnesium carbonate, silicates (for example, aluminum silicate, magnesium silicate, etc.), silicic acid, barium sulfate, calcium sulfate, calcium zincate, zinc oxide and titanium oxide.

As crosslinking agents there are preferred organic crosslinking agents including thermosetting resins (amino resins, phenol resins, epoxy resins, alkyd resins, unsaturated polyesters, etc.), isocyanate compounds and block isocyanate compounds, or inorganic crosslinking agents such as metals or metal compounds.

As antiseptics there are preferred ethyl paraoxybenzoate, propyl paraoxybenzoate and butyl paraoxybenzoate, and as ultraviolet absorbers there are preferred p-aminobenzoic acid derivatives, anthranilic acid derivatives, salicylic acid derivatives, coumarin derivatives, amino acid-based compounds, imidazoline derivatives, pyridine derivatives and dioxane derivatives.

As absorption accelerators there may be mentioned terpenes such as d-limonene, fatty acid esters such as glycerin monolaurate, glycerin monooleate and diethyl sebacate, azacycloalkanes such as azone and 1-[2-(decylthio)ethyl]azacyclopentan-2-one, and higher fatty acids such as oleic acid, lauric acid and myristic acid.

As other polymers there may be mentioned polymers containing unsaturated monomers with a total of 2-4 carbon atoms as the monomer unit (for example, polyisobutylene), and such polymers may be either tacky or non-tacky. When such a polymer component is added, the content thereof, based on the total weight of the adhesive, is 5-15 wt%, the total content of the base with tackiness and the oil is 80-90 wt%, and content of ketoprofen is preferably 0.5-3 wt% and more preferably 2 wt%. When polyisobutylene is used, it is preferred to use a combination of low molecular weight polyisobutylene with a viscosity-average molecular weight (Staudinger) of 10,000-20,000 (preferably 10,000-15,000) and high molecular weight polyisobutylene with a viscosity-average molecular weight (Staudinger) of 50,000-150,000 (preferably 60,000-120,000).

The adhesive may be used alone, but preferably it is spread on the support to form a drug layer for use as a plaster (adhesive with support). In this case, the drug layer may be formed on the support in an amount for coating of 80-210 g/m². The coating is preferably 100-200 g/m² and more preferably 120-180 g/m².

The support is not limited to a single-layer structure, and may be a laminated structure as well. For example, it may have a laminated structure comprising multi-layered woven fabrics (or knitted fabrics) or nonwoven fabrics made of different resin fibers. The woven fabrics or nonwoven fabrics may be formed of materials ordinarily used in plasters, and as preferred examples there may be mentioned woven fabrics and nonwoven fabrics composed of at least one type of resin fiber selected from the group consisting of polyester-based resins, polyethylene-based resins and polypropylene-based resins, among which woven fabrics made of the polyester-based polyethylene terephthalate are preferred because of low interaction with drugs.

The basis weight of the support is preferably 80-150 g/m², since such a range will allow satisfactory anchorage with the adhesive without bleeding of the adhesive through the openings of the support fabric network when the adhesive is coated onto the support.

In consideration of adequate stretching properties as a plaster, the support preferably has a 50% modulus of 2-12 N/5 cm in the lengthwise direction (long-axis direction) and a 50% modulus of 2-8 N/5 cm in the widthwise direction (short-axis direction). (Measuring method: JIS L1018)

A protective film may also be laminated on the drug layer for protection.

The adhesive has an area under the blood concentration-time curve at 0-72 hours (AUC₍₀₋₇₂ₕᵣ₎) of 1000-3000 ng·hr/mL for ketoprofen upon contact with skin. An AUC₍₀₋₇₂ₕᵣ₎ value in this range can produce a satisfactory anti-inflammatory analgesic effect. The area under the blood concentration-time curve for ketoprofen at 0-72 hours is more preferably 1200-2800 ng·hr/mL and even more preferably 1300-2500ng·hr/mL.

The adhesive also preferably has a maximum blood concentration (Cₘₐₓ) of 50-150 ng/mL for ketoprofen upon contact with skin. A Cₘₐₓ value in this range can produce a satisfactory anti-inflammatory analgesic effect. The maximum blood concentration is more preferably 60-150 ng/mL and even more preferably 65-140 ng/mL. The maximum blood concentration may be determined from blood samples taken after 72 hours of contacting the adhesive with the skin.

If the blood has AUC and Cₘₐₓ values in these ranges, the adhesive of the invention will exhibit a satisfactory anti-inflammatory analgesic effect even without addition of L-menthol.

The area (size) of the plaster is not restricted so long as it satisfies either or both of the following conditions (1) and (2).
(1) The area under the blood concentration-time curve at 0-72 hours (AUC₍₀₋₇₂ₕᵣ₎) is 1000-3000 ng·hr/mL for ketoprofen when the drug layer is contacted with skin.
(2) The maximum blood concentration (Cₘₐₓ) is 50-150 ng/mL for ketoprofen when the drug layer is contacted with skin.

However, from the standpoint of more effectively preventing skin irritation, the area of the drug layer to be contacted with the skin is preferably 50-150 cm², more preferably 60-80 cm² and most preferably 70 cm². The drug layer is preferably formed for 8-21 mg, more preferably 10-18 mg and especially 70 ±5 mg per 1 cm².

The adhesive and plaster may both be produced by publicly known processes. For example, since the adhesive can be obtained by stirring the base with tackiness, the oil and the ketoprofen in an organic solvent, it may be coated onto a support and the organic solvent removed to yield the plaster. Particularly if a thermoplastic elastomer-based compound is employed as the base with tackiness, the adhesive and plaster can be produced by a method of hot melt mixing without using an organic solvent.

### Examples

The present invention will now be explained in greater detail based on examples and comparative examples, with the understanding that these examples are in no way limitative on the invention. Unless otherwise specified, the "parts" refer to "parts by weight".

### (Example 1)

A plaster for Example 1 having the composition shown in Table 1 was prepared by the method described above. Specifically, the components other than ketoprofen in the aforementioned formulation were combined to form a mixture which was then heated and stirred under a nitrogen atmosphere to obtain a solution. Next, the ketoprofen was added to the solution and the mixture was heated and stirred to obtain a homogeneous solution. The solution was subsequently spread onto a support (polypropylene nonwoven fabric) to an adhesive layer thickness of 150 µm and then covered with a peel-off covering (polyester film), cooled and cut to a prescribed size to obtain a plaster.

### (Examples 2-6, Comparative Examples 1-3)

Plasters were prepared by the same production method as in Example 1, except that the compositions used were those for Examples 2-6 and Comparative Examples 1-3 shown in Table 1.

**(Table 1)**

| Composition (parts by weight) | | | | | | | | | | |
|---|---|---|---|---|---|---|---|---|---|---|
| | | Example 1 | Example 2 | Example 3 | Example 4 | Example 5 | Example 6 | Comp. Ex. 1 | Comp. Ex. 2 | Comp. Ex. 3 |
| Tacky composition | Elastomer [styrene-isoprene-styrene block copolymer (styrene/isoprene ratio: 22/78, diblock content: 18%, Solution viscosity: 1000 cps (25% toluene solution, 25 °C))] | 17.34 | 17.34 | 18.89 | 17.83 | 18.61 | 17.58 | 18.34 | 15.8 | 21.04 |
| | Tackifier [Hydrogenated rosin glycerin ester] | 16.01 | 16.01 | 14.53 | 13.71 | 15.75 | 14.88 | 16.93 | 20.3 | 9.16 |
| Oil | Liquid paraffin | 55.31 | 55.31 | 54.41 | 56.86 | 53.62 | 56.07 | 52.86 | 52.49 | 59.33 |
| Drug | Ketoprofen | 2 | 2 | 2 | 2 | 2 | 2 | 2 | 2 | 2 |
| Other Polymer | Polyisobutylene | 9.34 | 9.34 | 10.17 | 9.6 | 10.02 | 9.47 | 9.87 | 9.41 | 8.47 |

Table 2 shows the oil contents with respect to 100 parts by weight as the total of the elastomers and tackifiers (total of the tacky compositions) in the plasters of the examples and comparative examples.

**(Table 2)**

| Oil contents with respect to 100 parts by weight of tacky compositions (parts by weight) | | | | | | | | | |
|---|---|---|---|---|---|---|---|---|---|
| | Example 1 | Example 2 | Example 3 | Example 4 | Example 5 | Example 6 | Comp. Ex. 1 | Comp. Ex. 2 | Comp. Ex. 3 |
| Oil | 160.0 | 160.0 | 156.5 | 173.9 | 150.0 | 166.7 | 144.0 | 140.0 | 190.0 |

### [Measurement of area under the blood concentration-time curve and maximum blood concentration for ketoprofen]

The plaster of Example 1 (skin attachment drug layer area: 70 cm², 1 g coating thickness) was attached to healthy adults and peeled off after 24 hours, and blood samples were taken 72 hours after peel-off. Each blood sample was processed and the plasma ketoprofen concentration was measured by LC/MS. As a result, the area under the blood concentration-time curve (AUC₍₀₋₇₂ₕᵣ₎) in Example 1 was 1450 ng·hr/mL and the maximum blood concentration (Cₘₐₓ) was 77 ng/mL.

### [Plaster tack evaluation]

The plasters of Examples 2-6 and Comparative Examples 2-3 were evaluated for tack by the probe tack test method described below. As a result, as shown in Table 3, the plasters of Examples 2-6 exhibited satisfactory tack, while the plaster of Comparative Example 2 had higher tack than the plasters of Examples 2-6 and the plaster of Comparative Example 3 had lower tack than the plasters of Examples 2-6. It was therefore demonstrated that each of the plasters of the examples had satisfactory tack, and that varying the ratio of the tacky composition and oil can adjust the tack of the plaster to a suitable strength.

### <Probe tack test method>

A test piece was taken from the plaster, and using a probe tack tester according to ASTM D 2979, one base side of a 5 mm-diameter bakelite cylinder (probe) was contacted with the adhesive layer surface of the test piece at a contact load of 0.98 N/cm² for a contact time of 1.0 second, after which the probe was pulled away vertically from the adhesive layer surface at a speed of 5 mm/s and the force [gf] required at that time was measured. Five test pieces of the plaster were measured, and the average value was recorded as the probe tack value.

### [Plaster tack evaluation 2]

The plasters of Example 2 and Comparative Examples 2 and 3 were evaluated for tack by the peel test method described below. It was demonstrated that varying the ratio of the tacky composition and oil changed the tack (peel) of the plaster in a corresponding manner.

### <Peel test method>

The plaster was cut into a 20 mm-wide, approximately 100 mm-long test piece and adhered onto a phenol resin test board with a roller, and the load for peeling off at 180° at a speed of 300 mm/min with an Instron tensile tester was determined.

### [Evaluation of plaster adhesion]

The plasters of Example 2 and Comparative Examples 2-3 were evaluated for adhesion by the adhesion evaluation method described below. As a result, the plasters of Example 2 and Comparative Example 2 had satisfactory adhesion with no observable peel-off, while the plaster of Comparative Example 3 had an unsatisfactory adhesion, tending to peel from the skin around the outer edge sections.

### <Adhesion evaluation method>

The plaster was cut into a 10 cm-wide, 14 cm-long test piece and attached onto the inner forearm of a healthy adult for 6 hours, and then the adhered state of the plaster was observed and evaluated by the following criteria.
A: Satisfactory adhesion with no peeled sections.
B: Peeling at outer edges of plaster, unsatisfactory adhesion.

**(Table 3)**

| | Ex. 2 | Ex. 3 | Ex. 4 | Ex. 5 | Ex. 6 | Comp. Ex. 2 | Comp. Ex. 3 |
|---|---|---|---|---|---|---|---|
| Probe tack test (gf) | 65.4 | 68.8 | 64.0 | 71.8 | 68.0 | 95.2 | 52.2 |
| Peel test (N) | 0.44 | - | - | - | - | 0.58 | 0.34 |
| Adhesion | A | - | - | - | - | A | B |

### [Evaluation of skin stratum corneum detachment by plaster]

The plasters of Example 2 and Comparative Example 2 were each attached onto the inner forearm of a healthy adult for 6 hours and then peeled off, and then the stratum corneum (cells) adhering to the surface of the adhesive layer of the plaster were stained blue by the method described below and the proportion of stratum corneum adhering sections out of the total adhesion area was observed and evaluated. As a result, the plaster of Example 2 had comparatively little detachment of the stratum corneum from the skin, whereas the plaster of Comparative Example 2 had adhesion of stratum corneum from the skin over roughly the entire surface. In other words, the plaster of Example 2 had maintained the skin condition even after attachment, whereas the plaster of Comparative Example 2 produced detachment of the stratum corneum layer from the skin, thus potentially causing stimulation of the skin by use of the plaster.

### <Staining and evaluation method>

Following method described in Nitto Giho Vol. 28, No.1, p50-57, July 1990, the plaster peeled from the adult was immersed for 1 minute in dyeing solution (Gentian Violet 1.0%, Brilliant Green 0.5%, Distilled Water 98.5%) and then rinsed with distilled water, and the degree of detachment of the stratum corneum from the skin was evaluated based on the dyed color of the adhesive surface. An adhesive surface without stratum corneum adhesion is not dyed.

### [Evaluation of drug release from plaster]

The plasters of Examples 2-5 were evaluated for release of the drug (ketoprofen) by the release test with water described below. As a result, as shown in Table 4, the plasters of each of the examples were demonstrated to have satisfactory drug release properties.

### <Release test with water >

A test was carried out under the following conditions based on the rotary cylinder release test with water described in the U.S. Pharmacopeia Release Test. Specifically, the test piece was held in a cylinder under the conditions: test liquid = distilled water, liquid temperature = 37°C, cylinder bottom edge to vessel bottom inner surface = 12 mm, rotation = 50 rpm, and then the test liquid was sampled at 0 hr, 0.5 hr, 1.5 hrs and 4 hrs from the start of the test for HPLC quantitation of the amount of ketoprofen released and the release rate based on theoretical content was determined as a percentage (n=6).

**(Table 4)**

| | Release ratio (%) | | | | |
|---|---|---|---|---|---|
| Time (hr) | Example 2 | Example 3 | Example 4 | Example 5 | Example 6 |
| 0 | 0 | 0 | 0 | 0 | 0 |
| 0.5 | 26.5 | 24.9 | 25.4 | 22.6 | 24.3 |
| 1.5 | 46.9 | 46.6 | 47.8 | 42.6 | 46.0 |
| 4.0 | 80.2 | 75.9 | 77.1 | 70.7 | 74.7 |

As explained above, the adhesive and plaster of the invention are excellent as an adhesive and plaster having satisfactory skin adhesion properties, allowing skin irritation to be prevented and having high drug absorption efficiency.

## Claims

1. An adhesive comprising a base with tackiness, an oil and ketoprofen, wherein
said adhesive contains no L-menthol,
said base is composed at least of a tacky composition comprising an elastomer and a tackifier,
said oil is an oil that is compatible with said tacky composition, and the content of said oil is 150-175 parts by weight with respect to 100 parts by weight as the total of said tacky composition,
wherein said elastomer is a styrene-isoprene-styrene block copolymer with a styrene content of 15-25 wt% and a diblock content of no greater than 20 wt%, and wherein the mixing proportion of the tackifier is 50-200 parts by weight with respect to 100 parts by weight of the elastomer.

2. An adhesive according to claim 1, wherein said oil is at least one selected from the group consisting of almond oil, olive oil, camellia oil, persic oil, peanut oil and liquid paraffin.

3. An adhesive according to claim 1 or 2, wherein the ketoprofen content is 0.5-3 wt% based on the total weight of the adhesive.

4. An adhesive according to claim 1 or 2, wherein the ketoprofen content is 2 wt% based on the total weight of the adhesive.

5. An adhesive according to any one of claims 1 to 4, wherein the area under the blood concentration-time curve at 0-72 hours (AUC₍₀₋₇₂ₕᵣ₎) for ketoprofen upon contact with skin is 1000-3000 ng·hr/mL.

6. An adhesive according to any one of claims 1 to 4, wherein the maximum blood concentration (Cmax) for ketoprofen upon contact with skin is 50-150 ng/mL.

7. A plaster provided with a drug layer comprising an adhesive according to any one of claims 1 to 6 on a support.

8. A plaster according to claim 7, wherein said drug layer is formed on said support in an amount for coating of 80-210 g/m².

9. A plaster according to claim 7 or 8, wherein said support is made of polyester, polyethylene or polypropylene.

10. A plaster according to any one of claims 7 to 9, wherein said support is a woven fabric with a weight of 80-150 g/m².

## Patentansprüche

1. Klebstoff, umfassend einen Grundstoff mit Klebrigkeit, ein Öl und Ketoprofen, wobei
der Klebstoff kein L-Menthol enthält,
der Grundstoff wenigstens aus einer klebrigen Zusammensetzung zusammengesetzt ist, die ein Elastomer und einen Klebrigmacher umfasst,
das Öl ein Öl ist, welches mit der klebrigen Zusammensetzung verträglich ist, und der Gehalt des Öls 150 - 175 Gewichtsteile, bezogen auf 100 Gewichtsteile als die Gesamtmenge der klebrigen Zusammensetzung, beträgt,
wobei das Elastomer ein Styrol-Isopren-Styrol-Blockcopolymer mit einem Styrolgehalt von 15 - 25 Gew.-% und einem Diblockgehalt von nicht mehr als 20 Gew.-% ist und wobei das Mischungsverhältnis des Klebrigmachers 50 - 200 Gewichtsteile, bezogen auf 100 Gewichtsteile des Elastomers, beträgt.

2. Klebstoff gemäß Anspruch 1, wobei das Öl wenigstens eines, ausgewählt aus der Gruppe bestehend aus Mandelöl, Olivenöl, Kamelienöl, Pfirsichkernöl, Erdnussöl und flüssigem Paraffin, ist.

3. Klebstoff gemäß Anspruch 1 oder 2, wobei der Ketoprofengehalt 0,5 - 3 Gew.-%, bezogen auf das Gesamtgewicht des Klebstoffs, beträgt.

4. Klebstoff gemäß Anspruch 1 oder 2, wobei der Ketoprofengehalt 2 Gew.-%, bezogen auf das Gesamtgewicht des Klebstoffs, beträgt.

5. Klebstoff gemäß einem der Ansprüche 1 bis 4, wobei die Fläche unter der Blutkonzentration-Zeit-Kurve bei 0 - 72 Stunden (AUC₍₀₋₇₂ₕ₎) für Ketoprofen nach Kontakt mit der Haut 1000 - 3000 ng·h/ml beträgt

6. Klebstoff gemäß einem der Ansprüche 1 bis 4, wobei die maximale Blutkonzentration (Cmax) für Ketoprofen nach Kontakt mit der Haut 50 - 150 ng/ml beträgt.

7. Pflaster, das mit einer Arzneimittelschicht, die einen Klebstoff gemäß einem der Ansprüche 1 bis 6 umfasst, auf einem Träger versehen ist.

8. Pflaster gemäß Anspruch 7, wobei die Arzneimittelschicht auf dem Träger in einer Menge zur Beschichtung von 80 - 210 g/m² gebildet ist.

9. Pflaster gemäß Anspruch 7 oder 8, wobei der Träger aus Polyester, Polyethylen oder Polypropylen gefertigt ist.

10. Pflaster gemäß einem der Ansprüche 7 bis 9, wobei der Träger ein Gewebe mit einem Gewicht von 80 - 150 g/m² ist.

## Revendications

1. Adhésif comprenant une base présentant du collant, un huile et un cétoprofène, où
ledit adhésif ne contient pas de L-menthol,
ladite base est composée d'au moins une composition donnant du collant comprenant un élastomère et un agent donnant du collant,
ladite huile est une huile qui est compatible avec ladite composition donnant du collant, et la teneur de ladite huile est de 150 à 175 parties en poids par rapport à 100 parties en poids comme total de ladite composition donnant du collant,
où ledit élastomère est un copolymère séquencé de styrène-isoprène-styrène avec une teneur en styrène de 15 à 25 % en pds et une teneur en séquence double non supérieure à 20 % en pds, et où la proportion de mélange de l'agent donnant du collant est de 50 à 200 parties en poids par rapport à 100 parties en poids de l'élastomère.

2. Adhésif selon la revendication 1, où ladite huile est au moins l'une sélectionnée dans le groupe constitué de l'huile d'amande, de l'huile d'olive, de l'huile de camellia, de l'huile de noyau d'abricot, de l'huile d'arachide et de la paraffine liquide.

3. Adhésif selon la revendication 1 ou 2, où la teneur en cétoprofène est de 0,5 à 3 % en pds sur la base du poids total de l'adhésif.

4. Adhésif selon la revendication 1 ou 2, où la teneur en cétoprofène est de 2 % en pds sur la base du poids total de l'adhésif.

5. Adhésif selon l'une quelconque des revendications 1 à 4, où la surface sous la courbe de concentration dans le sang-temps à 0 à 72 heures (AUC₍₀₋₇₂ₕ₎) pour le cétoprofène à l'issue du contact avec la peau est de 1 000 à 3 000 ng•h/ml.

6. Adhésif selon l'une quelconque des revendications 1 à 4, où la concentration maximum dans le sang (Cmax) pour le cétoprofène à l'issue du contact avec la peau est de 50 à 150 ng/ml.

7. Emplâtre prévu avec une couche médicamenteuse comprenant un adhésif selon l'une quelconque des revendications 1 à 6 sur un support.

8. Emplâtre selon la revendication 7, où ladite couche médicamenteuse est formée sur ledit support en une quantité de revêtement de 80 à 210 g/m².

9. Emplâtre selon la revendication 7 ou 8, où ledit support est constitué de polyester, de polyéthylène ou de polypropylène.

10. Emplâtre selon l'une quelconque des revendications 7 à 9, où ledit support est un textile tissé présentant un poids de 80 à 150 g/m².
